# EUROPEAN PATENT SPECIFICATION

(11) **EP 3 936 852 B1**
(45) Date of publication and mention of the grant of the patent: **03.04.2024**
(21) Application number: 20766704.9
(22) Date of filing: 14.02.2020
(51) Int. Cl.: G01N 5/02, G01N 19/00, G01N 33/00

(54) **HYDROGEN SENSOR AND HYDROGEN DETECTION METHOD**
WASSERSTOFFSENSOR UND VERFAHREN ZUR WASSERSTOFFDETEKTION
CAPTEUR D'HYDROGÈNE ET PROCÉDÉ DE DÉTECTION D'HYDROGÈNE

(30) Priority: 06.03.2019 JP 2019040136
(43) Date of publication of application: 12.01.2022
(73) Proprietor: National Institute for Materials Science, Tsukuba-shi, Ibaraki 305-0047 (JP)
(72) Inventor: YAKABE, Taro, Tsukuba-shi, Ibaraki 305-0047 (JP); IMAMURA, Gaku, Tsukuba-shi, Ibaraki 305-0047 (JP); YOSHIKAWA, Genki, Tsukuba-shi, Ibaraki 305-0047 (JP); NAKAMURA, Akiko, Tsukuba-shi, Ibaraki 305-0047 (JP)
(74) Representative: Dehns
(86) International application number: PCT/JP2020/005694
(87) International publication number: WO 2020/179400

(56) References cited:
- EP-A1- 2 169 400
- WO-A1-2013/157581
- CN-A- 109 342 558
- JP-A- 2006 507 511
- JP-A- 2011 232 141
- JP-A- 2011 232 141
- JP-A- 2019 100 705
- KR-A- 20060 111 296
- US-A1- 2004 096 357
- PATTON J F ET AL: "Rapid response microsensor for hydrogen detection using nanostructured palladium films", SENSORS AND ACTUATORS A: PHYSICAL, ELSEVIER BV, NL, vol. 163, no. 2, October 2010 (2010-10), pages 464-470, XP027452616, ISSN: 0924-4247, DOI: 10.1016/J.SNA.2010.08.025 [retrieved on 2010-09-22]
- BASELT D R ET AL: "Design and performance of a microcantilever-based hydrogen sensor", SENSORS AND ACTUATORS B: CHEMICAL, ELSEVIER BV, NL, vol. 88, no. 2, 15 January 2003 (2003-01-15), pages 120-131, XP004399080, ISSN: 0925-4005, DOI: 10.1016/S0925-4005(02)00315-5
- GENKI YOSHIKAWA ET AL: "Nanomechanical Membrane-type Surface Stress Sensor", NANO LETTERS, vol. 11, no. 3, 9 March 2011 (2011-03-09), pages 1044-1048, XP055506585, US ISSN: 1530-6984, DOI: 10.1021/nl103901a
- KAJITA SUSUMU ET AL: "Composition Control of Pd-Cu-Si Metallic Glassy Alloys for Thin Film Hydrogen Sensor", MATERIALS TRANSACTIONS, vol. 51, no. 12, 2010, pages 2133-2138, XP055969006, JP ISSN: 1345-9678, DOI: 10.2320/matertrans.M2010254

## Description

### Technical Field

The present invention relates to a hydrogen sensor using a Membrane-type Surface stress Sensor (hereinafter referred to as MSS), and particularly to a hydrogen sensor using an amorphous palladium-copper-silicon alloy (hereinafter referred to as PdCuSi) thin film as a sensitive film thereof. The present invention also relates to a method for detecting hydrogen using such a hydrogen sensor.

### Background Art

In order to reduce environmental load, for example, research and development for utilization of hydrogen as energy sources has been advanced, and practical utilization has been achieved in some cases. One problem in the wide utilization of hydrogen is a possibility of explosion due to the leakage of hydrogen from a container, piping, or the like. Since hydrogen is light and diffusive, in an open space, hydrogen is less prone to accumulate and it can be said that the risk of explosion is not so high. On the other hand, when the leakage of hydrogen occurs in a nearly closed compartment, the retention of hydrogen may occur. When hydrogen is retained and the hydrogen concentration in the air enters a wide region (explosion range) of 4 to 75 volume % (hereinafter, hydrogen concentration is expressed by a volume ratio unless otherwise specified), ignition causes explosion. Therefore, it is very important to discover the leakage or accumulation of hydrogen at an early stage while the hydrogen concentration is sufficiently lower than 4% in view of ensuring the safety of hydrogen-related equipment or facilities.

Hydrogen sensors based on various operation principles proposed so far include those in which a thin film of a hydrogen storage material is formed on one side of a micro cantilever and the hydrogen concentration in a sample gas is measured from a change in the amount of deflection of the cantilever (displacement of the cantilever end) caused by the expansion and contraction of the thin film due to the storage and release of hydrogen by the thin film (for example, Non Patent Literature 1 and Non Patent Literature 2). This type of sensor (cantilever-type sensor) can selectively detect specific substances in a liquid or gas sample by appropriately selecting a film (referred to as a sensitive film, receptor layer, or the like) of a substance adhered to the surface of the cantilever, and thus various applications thereof have been proposed for detecting trace components.
Patton et al. (Sensors and Actuators A 163 (2010) 464-470) describe a rapid response microsensor for hydrogen detection using nanostructure palladium films. JP2011/232141 describes a hydrogen sensor in which a hydrogen sensitive body is formed on a resin substrate.

Since hydrogen has a risk of explosion even at a low concentration of 4% as described above, it is required to increase the sensitivity of the hydrogen sensor as possible in order to detect the accumulation of hydrogen due to leakage or the like sufficiently prior to its concentration reaches the explosion range. Particularly, in a closed region having a complex internal structure or shape, the distribution of hydrogen concentration in the region may largely change due to a position where the leakage of hydrogen occurs, and thus the sensitivity of the hydrogen sensor is required to have a further allowance in order to deal with such a case. However, since about 0.5 ppm of hydrogen is typically contained in the air (Non Patent Literature 3), such a high sensitivity that can detect a value close to 0.5 ppm is not necessary in use for the purpose of detecting a small amount of hydrogen leakage.

US 2004/096357 discloses a related hydrogen sensor where the sensitive film is a palladium thin film and whereby a surface stress results in an out of plane deflection of the membrane.

The inventors of the present application have found that the above-mentioned objective is achieved by an MSS using a thin film of a hydrogen storage material such as a hydrogen storage metal or alloy such as palladium having a thickness or 30 nm or less as a sensitive film, and a patent application has been filed as Japanese Patent Application No. 2017-155808 (JP 2019-35613 A).

### Summary of Invention

### Technical Problem

While the hydrogen sensor for which the patent application has already been filed has a feature of being capable of detecting hydrogen at a sufficient high sensitivity and capable of hydrogen detection in the absence of oxygen, it is desirable to improve the S/N ratio of measurement data and the stability of measurement results particularly in a low concentration range by further improving the sensitivity. In addition, with typical hydrogen storage materials, the detection speed is low due to slow storage and release of hydrogen, and it is rather difficult to handle response signals due to hysteresis characteristics of storage and release, for which improvements are desired.

### Solution to Problem

According to an aspect of the present invention, a hydrogen sensor having a sensitive film on a surface for receiving surface stress of a membrane-type surface stress sensor is provided, wherein the sensitive film is an amorphous palladium-copper-silicon alloy thin film.

Here, an atomic ratio between palladium, copper, and silicon contained in the amorphous palladium-copper-silicon alloy may be 65 < x < 90, 3 < y < 20, and 3 < z < 20 wherein the alloy is represented as PdₓCu_{y}Si_{z}, with x, y, and z being percentage values.

The sensitive film may have a thickness of greater than 0 nm and less than 100 nm.

The sensitive film may have a thickness of 50 nm or less.

The sensitive film may have a thickness of 15 nm or more.

The sensitive film may have a thickness of 5 nm or more.

According to another aspect of the present invention, a method for detecting hydrogen is provided, comprising alternately supplying a target gas containing hydrogen and a purge gas to any of the hydrogen sensor above and measuring a hydrogen concentration in the target gas based on an output signal from the hydrogen sensor.

Here, an arithmetic treatment may be performed on the output signal.

The arithmetic treatment may be time differentiation.

In addition, a process of calculating the hydrogen concentration may be performed based on a peak value of the output signal on which the time differentiation has been performed.

Alternatively, a process of obtaining the hydrogen concentration may be performed based on a signal waveform following a peak value of the time differentiated output signal.

The process of calculating the hydrogen concentration based on the signal waveform following the peak value of the time differentiated output signal may be selectively performed based on the peak value of the time differentiated output signal.

### Advantageous Effects of Invention

The present invention provides a hydrogen sensor having a high sensitivity and low hysteresis characteristics while having a simple configuration and low cost, and a method for detecting hydrogen using the hydrogen sensor.

### Brief Description of Drawings

Fig. 1 is a schematic view of a Membrane-type Surface stress Sensor (MSS).
Fig. 2 is a photograph of a hydrogen sensor of an example of the present invention formed by depositing an amorphous PdCuSi ternary film on a substrate of the MSS by sputtering.
Fig. 3 is a schematic configurational diagram of a hydrogen measurement apparatus configured by using the hydrogen sensor of the example of the present invention.
Fig. 4 is a diagram showing changes in the output voltage of the MSS when a hydrogen sensor provided with an amorphous PdCuSi thin film having a thickness of 30 nm is used in the hydrogen measurement apparatus shown in Fig. 3 and, while constantly supplying a gas at 25°C, a cycle of changing its hydrogen concentration from no hydrogen to 0.2%, 0.48%, 1%, 2%, 4%, 2%, 1%, 0.48%, and 0.2% in this order every 300 seconds is repeated six times.
Fig. 5 is a diagram showing changes in the output voltage of the MSS when a hydrogen sensor provided with an amorphous PdCuSi thin film having a thickness of 30 nm is used in the hydrogen measurement apparatus shown in Fig. 3 and, while constantly supplying a gas at 25°C, a cycle of switching the hydrogen concentration in the gas between no hydrogen and 0.2% every 300 seconds is repeated three times, and cycles in which the hydrogen concentration at the time of containing hydrogen is set to 0.48%, 1%, 2%, and 4%, respectively, are each repeated three times.
Fig. 6 is a diagram showing changes in the output voltage of the MSS when a hydrogen sensor provided with an amorphous PdCuSi thin film having a thickness of 30 nm is used in the hydrogen measurement apparatus shown in Fig. 3 and, while constantly supplying a gas at 25°C, a cycle of switching the hydrogen concentration in the gas between no hydrogen and 12 ppm every 30 minutes is repeated twice, and cycles in which the hydrogen concentration at the time of containing hydrogen is set to 25 ppm, 50 ppm, and 100 ppm, respectively, are each repeated twice.
Fig. 7 is a diagram obtained by plotting peak values of the output voltage of the MSS shown in Fig. 5 and 6 with respect to the 1/2-th power of concentration. Further, in this measurement, the same experiment is performed for a hydrogen sensor provided with an amorphous PdCuSi thin film having a thickness of 50 nm. Saturation values at thicknesses of 30 nm and 50 nm are indicated by solid squares and open circles, respectively.
Fig. 8 is a diagram showing changes in the output voltage of the MSS when a hydrogen sensor provided with an amorphous PdCuSi thin film having a thickness of 30 nm is used in the hydrogen measurement apparatus shown in Fig. 3 and, while constantly supplying a gas at 25°C, a cycle of switching the hydrogen concentration in the gas between no hydrogen and 0.25 ppm every 300 seconds is repeated three times, and cycles in which the hydrogen concentration at the time of containing hydrogen is set to 0.5 ppm, and 1 ppm, respectively, are each repeated three times.
Fig. 9 is a diagram showing the result of numerical differentiation of the output voltage of the MSS shown in Fig. 8 with respect to time.
Fig. 10 is a diagram showing the result of numerical differentiation of the output voltage of the MSS shown in Fig. 5 with respect to time.
Fig. 11 is a diagram showing the result of numerical differentiation of the output voltage of the MSS shown in Fig. 6 with respect to time.
Fig. 12 is a diagram showing a result of plotting the peak values for each hydrogen concentration from Figs. 9, 10, and 11 obtained by differentiation with respect to concentration.
Fig. 13 is a diagram showing data of the hydrogen sensor of the example of the present invention in a comparative experiment of the hydrogen sensor of the example of the present invention and a hydrogen sensor using a Pd film as a sensitive film.
Fig. 14 is a diagram showing data of the hydrogen sensor using the Pd film as the sensitive film in the comparative experiment of the hydrogen sensor of the example of the present invention and the Pd film hydrogen sensor.

### Description of Embodiments

As a result of intensive studies, the inventors of the present application have found that an MSS using an amorphous PdCuSi thin film as a sensitive film achieves the above-mentioned objective, and brought the present invention into completion.

Note that it is conventionally known that an amorphous Pd-based alloy exhibits a hydrogen storage property, and hydrogen sensors using this property have been proposed. For example, Patent Literatures 4 to 6 and Non Patent Literature 4 disclose hydrogen sensors that use change in electrical resistance of amorphous PdCuSi. In addition, Patent Literature 7 and Non Patent Literature 5 disclose hydrogen sensors that use change in capacitance caused by the displacement of a PdCuSi film due to the film bending when storing hydrogen. However, since such hydrogen sensors using change in electrical resistance involve passing currents in PdCuSi, there is a problem of high power consumption during operation of the sensor. In addition, hydrogen sensors using change in capacitance as described above require a stacking structure of PdCuSi film in a direction perpendicular to the film surface, which leads to complexity in structure and difficulty in manufacture. In addition, it is difficult to reduce the thickness of a PdCuSi film in terms of its structure (according to the left column of page 316 in Non Patent Literature 5, the thickness of the PdCuSi film is 500 nm), and thus the amount of use of Pd, which is an expensive noble metal, is increased.

The present invention provides a hydrogen sensor that exhibits a high sensitivity and relatively good response characteristics even with an amorphous PdCuSi thin film having a very small thickness, has a low hysteresis and a simple structure, and can reduce power consumption by using an MSS as a surface stress sensor.

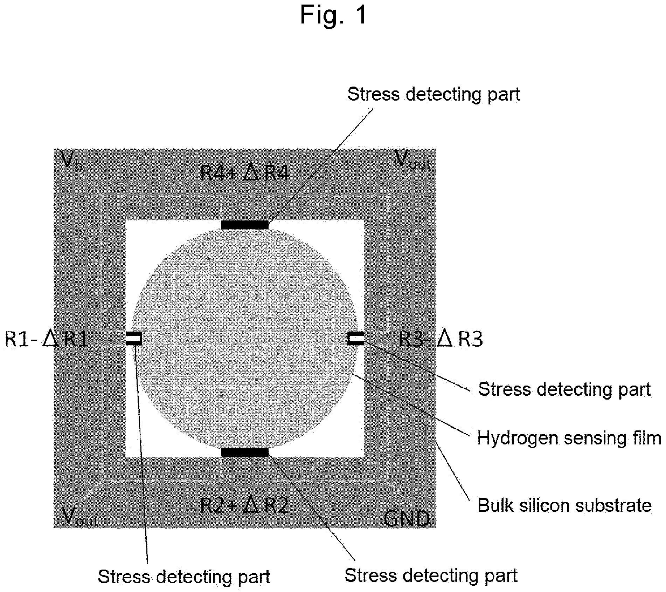

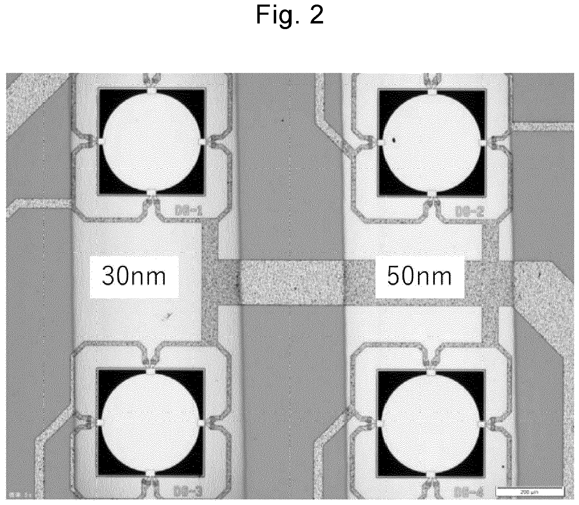

First, the MSS will now be described. As seen from the schematic diagram shown in Fig. 1, in the MSS, four peripheral portions of a thin plate-shaped member having a disc shape (which may alternatively be a shape having symmetry of rotation by an integer multiple of 90 degrees about its center, such as a square shape) is supported from outside (by a bulk silicon substrate in Fig. 2), instead of coating a sensitive film onto a surface of a rectangular base extending in a longitudinal direction. These four support portions are provided at positions of rotational symmetry at 90 degrees from the center of the member (that is, if the thin plate-shaped member is rotated by 90 degrees about the center point of the member, an adjacent support portion comes to the same position as a support portion located there before the 90 degrees rotation). Surface stress generated in the surface of the thin plate-shaped member is concentrated at these four support portions, and thus amplified uniaxial stress is applied to these support portions. This stress causes a change in electrical properties (electrical resistance in a currently manufactured MSS) of stress detecting parts provided at the respective support portions, which enables the surface stress to be detected. Further, as shown in Fig. 1, resistances R1 - ΔR1, R2 + ΔR2, R3 - ΔR3, and R4 + ΔR4 of the four stress detecting parts are connected in a full Wheatstone bridge configuration, and the voltage that appears between two Vₒᵤₜ terminals when a voltage is applied between two, V_{b} and GND terminals is taken out as an output of the MSS. The thin plate-shaped member and its peripheral portions are typically made from a silicon wafer, and the stress detecting parts are typically realized by forming piezo-resistive elements at corresponding portions of the silicon wafer.

In the MSS, in detecting surface stress in the surface of the thin plate-shaped member (sensor body surface), two orthogonal components of surface stress generated on the sensor body surface by the sensitive film (mentioned as "hydrogen sensing film" in Fig. 1, reflecting the subject matter of the present invention) and having components in any directions are separately detected by two pairs of stress detecting parts at positions of 90 degrees rotation relative to each other, instead of only detecting surface stress in one particular direction (the longitudinal direction of the cantilever) as in a cantilever-type sensor. In this manner, all directional components of the surface stress on the sensor body surface can be utilized for the detection thereof, and the stress on the surface of the thin plate-shaped member is concentrated at the narrowed portions serving as the detection units, and thus a good detection efficiency can be achieved. Further, since outputs (resistance changes in the case of Fig. 1) of the four stress detecting parts are brought together into one output (voltage appearing between the Vₒᵤₜ terminals) by connecting them in a full Wheatstone bridge configuration, an output with a large amplitude can be obtained, and common phase noise components contained in the outputs of the four stress detecting parts can be cancelled, so that S/N is improved. Thus, it is expected for the MSS to have a higher sensitivity than a cantilever-type sensor using equivalent materials by about 130 times at the maximum (Non Patent Literatures 6 and 7).

Types of cantilever-type sensors currently used include light reading cantilevers and piezo-resistive cantilevers, and in comparison for sensitivity, MSS ≥ light reading cantilevers >> piezo-resistive cantilevers. The reason for which an MSS even using a piezo-resistive element as a stress detecting member can have a much higher sensitivity than piezo-resistive cantilevers is that it is possible to utilize the fact that, in the case of using a (001) face of a p-type silicon monocrystal for the stress detecting members (and the thin plate-shaped member formed integrally with them) of the MSS, piezo-resistivities when current flows on this surface in the [110] direction have opposite signs in the [110] and [1/10] directions (where "/1" indicates a symbol of 1 with an overbar). That is, in this case, assuming a coordinate system where the above-mentioned two directions are set as x and y axes, an infinitesimal change dR in a piezo-resistance value R is proportional to the difference, σₓ - σ_{y}, between an x-direction stress and a y-direction stress. Thus, by configuring the four stress detecting members such that currents flow in the same direction ([110] direction) in the piezo-resistive elements of these stress detecting members (note that the black bold lines indicated at the four stress detecting parts in the MSS structure shown in Fig. 1 are all in the horizontal direction, i.e., the [110] direction), when surface stress is applied to the thin plate-shaped member, the piezo-resistance values of the stress detecting members adjacent to each other around it change in the opposite directions (note that the signs of the resistance changes ΔR1 to ΔR4 of the stress detecting members shown in Fig. 1 change alternately as -, +, -, and + counterclockwise in the order from ΔR1), and as a result, a large output change is obtained from the full Wheatstone bridge formed by these piezo-resistive elements. For a detailed analysis of this, see Non Patent Literature 6, for example.

The MSS is well known to those skilled in the fields of surface stress sensors and their applications, and will not be described in more detail. If more detailed information is required, see Patent Literatures 1 to 3 and Non Patent Literature 6, which describe the MSS, for example.

In the hydrogen sensor of the present invention, a thin film of amorphous PdCuSi, which is a kind of hydrogen storage material, is used as the sensitive film of the MSS. As described in the following example, examining the relationship between a gas (mixture gas of hydrogen and nitrogen) with a very wide range of hydrogen concentration from 4% on the high-concentration side to 0.25 ppm on the low-concentration side, which is even 1/2 of the concentration of hydrogen typically contained in the air, about 0.5 ppm, as described above, and the detection output of the MSS, an effect beyond expectation is obtained showing that, even in the case of using an amorphous PdCuSi thin film with a very small thickness of less than 100 nm, specifically 30 nm, which is in a range for which inspection has not been made in Non Patent Literatures 4 and 5 or the like, it is possible to detect a very low concentration of hydrogen at the lower limit of this range with a sufficiently high S/N ratio. Although the example also involves an experiment for the case of using an amorphous PdCuSi thin film having a thickness of 50 nm as the sensitive film, as can be understood theoretically and from the experiment results of the example, the intensity of an output signal is smaller as the thickness is smaller in a thickness range of this level, and therefore the experiment of the example is performed by mainly using the amorphous PdCuSi thin film having the smaller 30 nm thickness, which imposes stricter conditions. Although an experiment for the case where the amorphous PdCuSi thin film is thinner than 30 nm is not performed, the sensitivity does not largely change due to the thickness in a range of thickness of a few tens of nm, as discussed in Non Patent Literature 9, and therefore the sensitivity does not become ten times or 1/10 even when the thickness becomes twice or half, for example. Therefore, considering the fact that hydrogen at a concentration of 0.25 ppm can be detected with a sufficiently high S/N ratio when the thickness is 30 nm, it is considered that hydrogen to a concentration of about 0.25 ppm can be sufficiently detected even when the thickness is decreased to about 20 nm, and if the detection of a concentration of about 0.5 ppm is required, it is possible to further reduce the thickness to about 15 nm.

If a further lower detection sensitivity is allowed, it is possible to further reduce the thickness. However, depending on the deposition method and deposition conditions of the PdCuSi thin film, if the average thickness is reduced to about 5 nm, there is a tendency that the actually formed film would be in a state that is difficult describe as a film in a usual meaning such as minute particles scattered on the base or a large number of minute island-like regions discontinuous with each other formed on the base at spaces. Since in such a state, surface stress generated on the detector body surface due to storage and release of hydrogen by the thin film is largely decreased as compared to the case of considering a model in which a continuous and uniform film is formed on the base, it is considered that a continuous and uniform film results in a higher sensitivity of hydrogen detection. In this context, it can be said that the lower limit of the thickness of the sensitive film is about 5 nm unless a special film formation technique or the like is used to form a highly continuous and very thin film. On the other hand, as can be understood from Non Patent Literature 8, it is considered that a significant reduction in sensitivity of the surface stress sensor does not occur simply because of discontinuity of the sensitive film. Thus, not only a completely continuous film but also a discontinuous film with breaks or the like is allowed as the sensitive film, and in this context, it should be understood that a thickness mentioned herein refers to an average thickness. According to the same literature, reduction in sensitivity becomes significant when a state where small regions such as island-like or particulate are distributed at spaces from each other in a discontinuous minute structure of the sensitive film, that is, a ratio of coverage of the sensitive film on the sensor body surface is substantially lower than 1.

In addition, as described in the following example, although the storage of hydrogen is a phenomenon that generally requires relatively long time despite the fact that the storage of hydrogen by PdCuSi is more rapid than other hydrogen storage metals, if the sensitive film of hydrogen storage material is formed to be very thin, the amount of storage of hydrogen in the sensitive film approaches a state of equilibrium in a short time when the supply of hydrogen-containing gas (sample gas) to be measured to the MSS is started. Thus, it is possible to detect the hydrogen concentration in the sample gas with a high accuracy by using output signals from the MSS only at the beginning of the supply of the sample gas and in its vicinity. Although no limitation is intended of course, it is possible to easily extract hydrogen concentration information included in such signals near the beginning of the supply by performing operations such as time differentiation on the output signal from the MSS. More specifically, it is possible to determine the hydrogen concentration with a high accuracy only by observing peak values of the time derivative of the output signals from the MSS. In addition, there is a tendency that the storage of hydrogen slowly reaches equilibrium when the hydrogen concentration is particularly low, and in that case, it is difficult to determine the hydrogen concentration with a high accuracy only from the peak values. Even in such a case, differences in hydrogen concentration are reflected in waveforms and values (hereinafter referred to as "waveforms and the like") of output signals while some length of time elapses from the beginning of the supply of the target gas or of those after operations such as time differentiation. Therefore, it is possible to determine the hydrogen concentration with a high accuracy even in a low concentration range by using information of such waveforms and the like. In addition, it is also possible to improve the detection accuracy in a low hydrogen concentration range by performing division into cases according to hydrogen concentration (specifically, peak values obtained, for example) and, when the detected hydrogen concentration is low, determining the hydrogen concentration by additionally considering information of waveforms and the like as described above, and to determine the hydrogen concentration in a short time by using only the peak values in a range where the hydrogen concentration can be obtained with a high accuracy without such information.

In the example, an example of amorphous PdCuSi having a composition where the atomic ratios of Pd:Cu:Si are 75%:10%:15% is used; however, the present invention is not limited thereto. Based on the triangular phase diagram in Non Patent Literature 4 and Non Patent Literature 5, the range of atomic ratios in which PdCuSi can take an amorphous structure is represented by PdₓCu_{y}Si_{z}, where x, y, and z are expressed by percentage as 65 < x < 90, 3 < y < 20, and 3 < z < 20.

### Examples

In the following, a hydrogen sensor in which a thin film (with thicknesses of 50 nm and 30 nm as shown in Fig. 2) using PdCuSi (specifically, Pd75Cu10Si15) as a hydrogen storage material is formed on a flat member for receiving surface stress (circular portions shown at the center of Fig. 1, the upper left, lower left, upper right, and lower right of Fig. 2) in a membrane-type surface stress sensor (MSS), whose conceptual structure is shown in Fig. 1 and whose optical microscopic photograph is shown Fig. 2, is used as an example, and it is shown that this hydrogen sensor has a high sensitivity through measurement of characteristics of the hydrogen sensor. Note that, as a matter of course, the present invention is not limited to the specific hydrogen sensor used in the example, and the technical scope thereof is solely defined by each claim in the claims of the present application.

On the MSS, Pd75Cu10Si15 was deposited onto the flat member for receiving surface stress by ternary simultaneous sputtering. As described above, two thicknesses of 30 nm and 50 nm are illustrated in Fig. 2. PdCuSi is an amorphous hydrogen storage alloy and is known as a material having low hysteresis in storage and release.

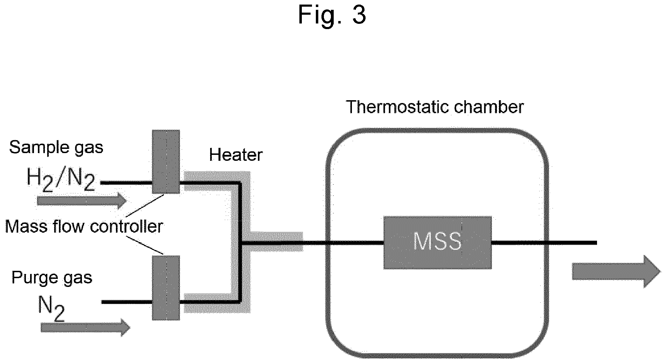

Fig. 3 shows an outline of an experiment system used in the example. A sample gas of hydrogen (or a mixture gas of hydrogen and nitrogen at known concentrations) was diluted with nitrogen to examine the response (output voltage) of the MSS at each hydrogen concentration. Here, the MSS with the deposited sensitive film was placed in a thermostatic chamber under temperature control, and gas was introduced from outside. The hydrogen concentration of the gas can be changed by control by a flowmeter (mass flow controller, MFC). The inflow gas and the interior of thermostatic chamber were set at the same temperature. In this example, measurement was performed at 25°C.

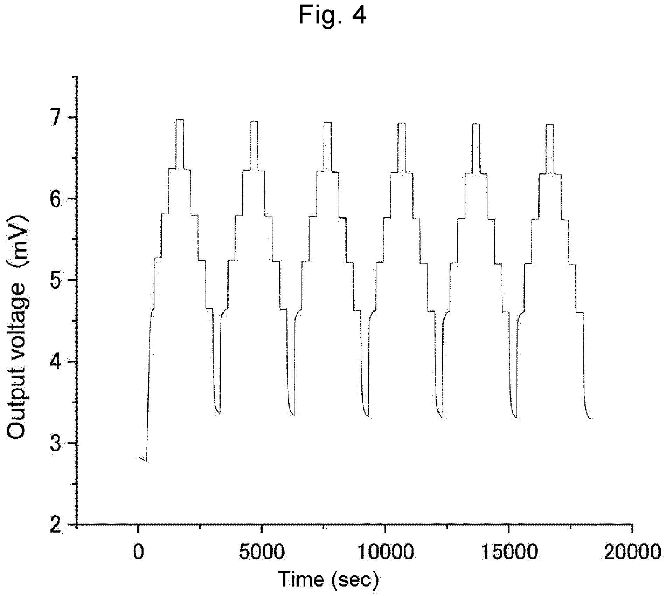

Fig. 4 shows the output voltage from the hydrogen sensor consisting of the MSS using the amorphous PdCuSi thin film having a thickness of 30 nm as a sensitive film. Here is shown the output voltage of the hydrogen sensor when the hydrogen concentration was changed from 0% to 0.2%, 0.48%, 1%, 2%, 4%, 2%, 1%, 0.48%, and 0.2% every 300 seconds and this cycle was repeated six times. Considering the fact that the same output voltage value is exhibited during an increase and decrease in concentration at the same concentration, it can be seen that this hydrogen sensor has almost no hysteresis. Thus, for example, a signal waveform in an increasing process of hydrogen concentration and a signal waveform in a decreasing process are substantially in a mirror relationship with each other, and one signal waveform contains almost all response information, and therefore it is possible to simplify the processing on the signal waveforms. Furthermore, comparing these signal intensities with results of using simple Pd as a sensitive film in Japanese Patent Application No. 2017-155808, already filed by the applicant of the present application, larger output voltage values are obtained in this example. Details will be described later.

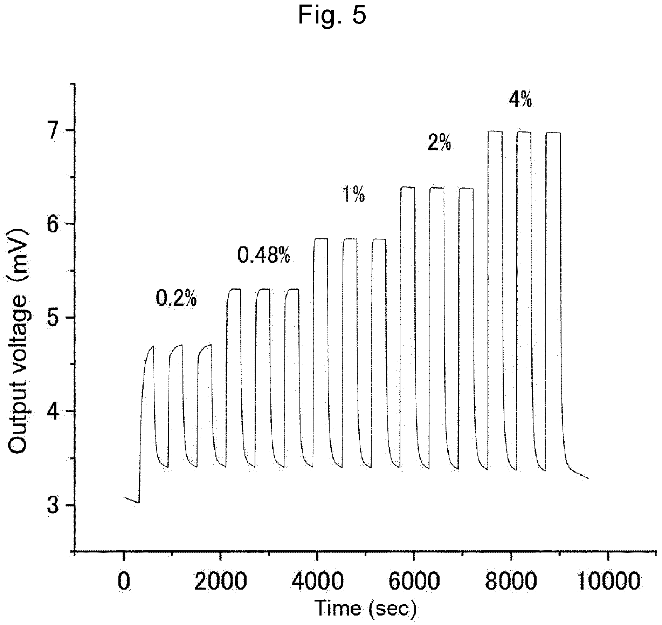

Fig. 5 shows the output voltage of the hydrogen sensor when measurement in which a cycle of switching the hydrogen concentration between one hydrogen concentration of 0.2%, 0.48%, 1%, 2%, and 4% and 0% every 300 seconds was repeated three times and then the same switching was performed for another hydrogen concentration was repeated under the same conditions as in Fig. 4 for the temperature of the gas and the thickness of the amorphous PdCuSi thin film. Saturation values of output voltage were approximately the same at the same hydrogen concentration, and the time to reach saturation was about 10 seconds at the hydrogen concentration of 4%.

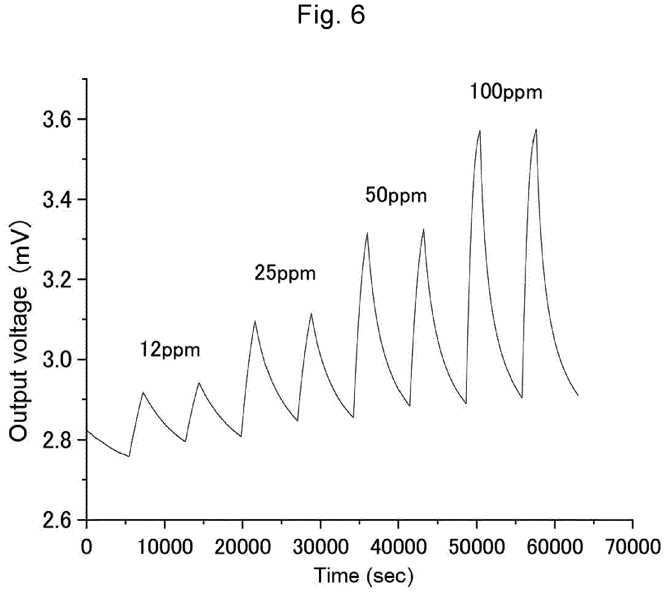

Fig. 6 shows the output voltage of the hydrogen sensor when measurement similar to that in Fig. 5 was performed under the same conditions as in Fig. 5 for the temperature of the gas and the thickness of the amorphous PdCuSi thin film. However, the hydrogen concentration was set to low concentrations, specifically, 12 ppm, 25 ppm, 50 ppm, and 100 ppm, and the number of repetitions of the above-mentioned cycle at the same hydrogen concentration was set to two. A cycle of supplying the hydrogen-containing gas for 30 minutes at each hydrogen concentration and supplying only nitrogen for 90 minutes was repeated twice at each hydrogen concentration. It can be seen from this result that the time until the output voltage of the hydrogen sensor reaches saturation is longer when the hydrogen concentration is lower.

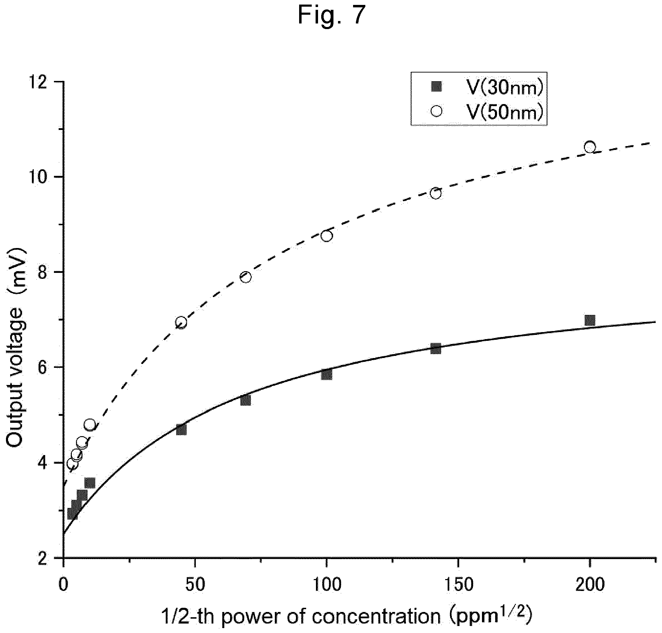

Fig. 7 shows results of plotting peak values of the output voltage of the hydrogen sensor with respect to the 1/2-th power of various hydrogen concentrations of the gas in two cases of 30 nm and 50 nm for the thickness of the amorphous PdCuSi thin film under the same condition as in Fig. 5 for the temperature of the gas. As can be seen from this figure, output values (peak values) have a positive correlation with concentration. In addition, as a matter of course, it can also be seen that, if the thickness of the amorphous PdCuSi thin film used as the sensitive film is made very small as above, the output voltage is lower when the thickness is smaller.

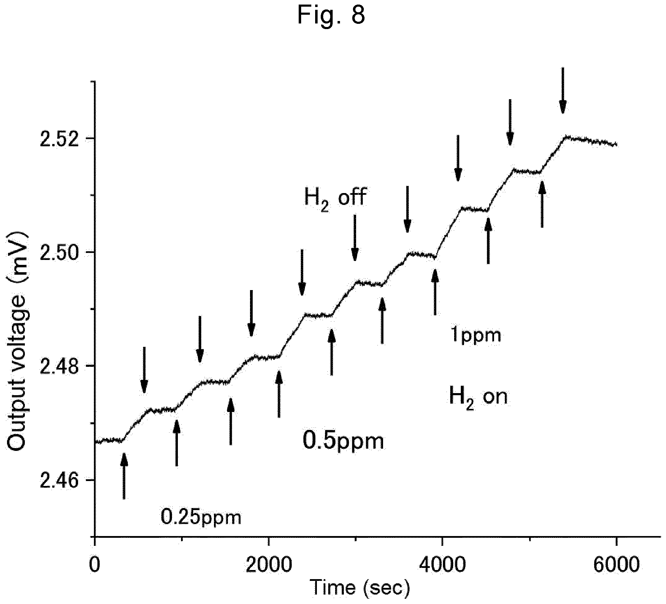

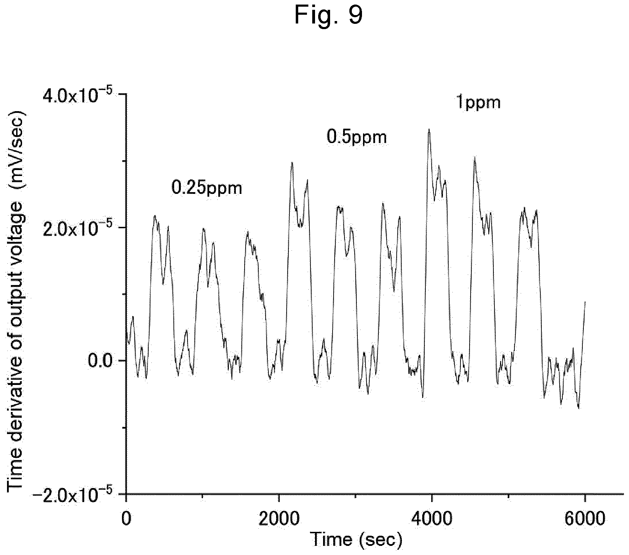

Fig. 8 shows the output voltage of the hydrogen sensor when the temperature of the gas was under the same condition as in Fig. 5, the thickness of the amorphous PdCuSi thin film was 30 nm, and the hydrogen gas concentration was further lower, specifically 0.25 ppm, 0.5 ppm, and 1 ppm. Here, gas was supplied to the hydrogen sensor for 300 seconds for each hydrogen concentration, and it was impossible to make determination with saturation values because it takes a long time to reach a state of saturation. However, it can be seen from changes in output voltage shown in the figure that the slopes of these changes (that is, the above-mentioned waveforms) differ according to the hydrogen concentration. Fig. 9 shows the time derivative of this graph.

Fig. 9 is the time derivative of the output voltage of the hydrogen sensor when the hydrogen concentration was 0.25 ppm, 0.5 ppm, and 1 ppm. Constant signals can be obtained at each hydrogen concentration. The Savitzky-Golay (SG) method, which is a method of approximating n adjacent points by a polynomial and then calculating its derivative, was used for the time differentiation. In Fig. 9, the calculation was performed by using quadratic approximation and 101 adjacent points.

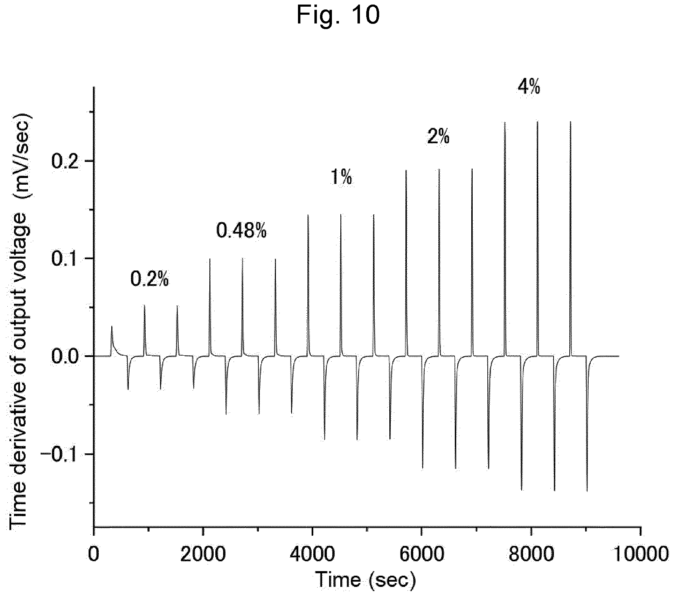

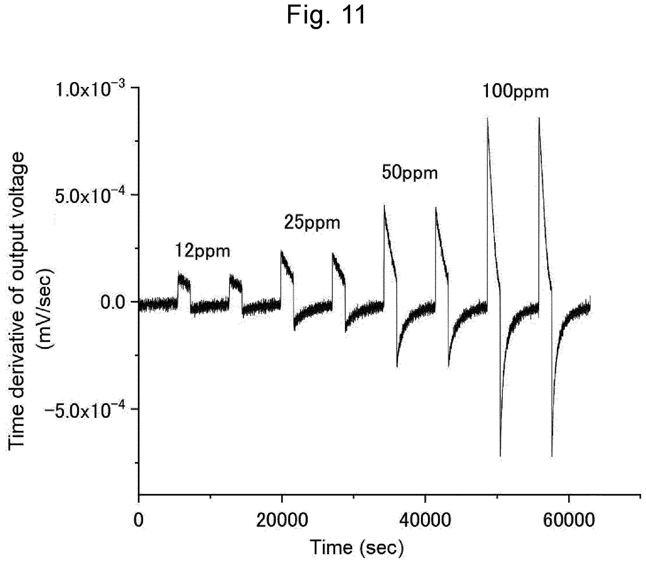

Figs. 10 and 11 show the time derivatives of the output voltages shown in Figs. 5 and 6, respectively. It can be seen that the derivative values differ according to concentration. The derivative values of the signals are considered as quantities corresponding to the speed of storage of hydrogen into the sensitive film, and represent differences in the storage speed with respect to the partial pressure of hydrogen in a hydrogen atmosphere. Figs. 10 and 11 are obtained by numerical differentiation using the SG method as in Fig. 9, and are calculated by using quadratic approximation and 21 adjacent points. A smaller number of adjacent points are used than in the case of Fig. 9 because of the fact that signal voltages have higher intensities at a higher concentration and the difference in S/N ratio.

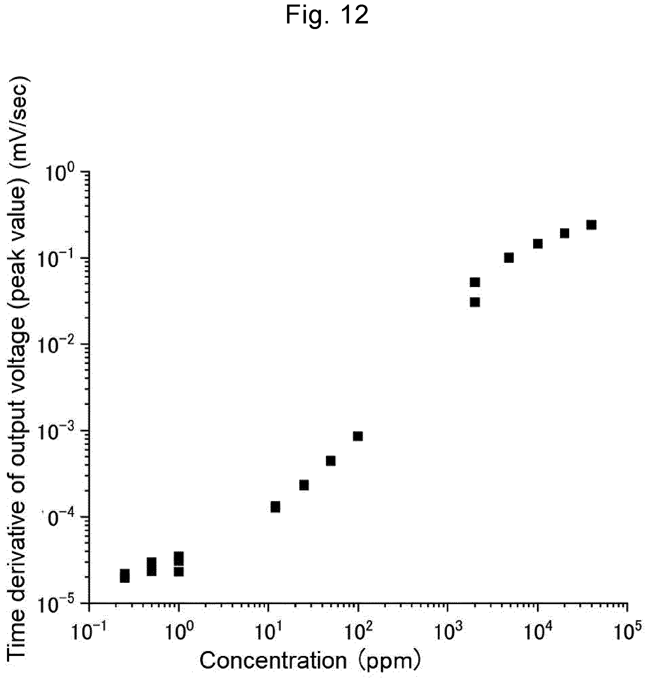

Fig. 12 plots peak values at each concentration from Figs. 9, 10, and 11 with respect to concentration. These peak values have a tendency of monotone increase with respect to concentration. Thus, it is possible to determine the presence and concentration of hydrogen at a low concentration of 1 ppm or less by performing time differentiation on the output voltage of the hydrogen sensor of the present invention. Specifically, even when the hydrogen concentration is 0.25 ppm, the derivative values of the output voltage of the hydrogen sensor of the present invention while such low concentration gas is provided can be easily distinguished from the derivative values in other states. Therefore, by using the hydrogen sensor of the present invention, it is possible to detect gas even with a very low hydrogen concentration of 0.25 ppm with a sufficiently high S/N ratio without special signal processing, as can be seen from Figs. 11 and 12. Furthermore, this example only shows an experiment on gas with a hydrogen concentration of 0.25 ppm. Although an experiment for 0.12 ppm was performed, it is considered that about 0.25 ppm is the detection limit under the thickness conditions of 30 nm to 50 nm. There is a possibility that a further lower concentration can be measured by using a thicker film.

Here, an experiment was conducted for specifically comparing the sensor operations of the hydrogen sensor composed of the MSS using the amorphous Pd75Cu10Si15 thin film (30 nm thick) sensitive film in this example and the hydrogen sensor composed of the MSS using the Pd thin film (20 nm thick) in Japanese Patent Application No. 2017-155808 described above. Although the Pd thin film has a smaller thickness, the Pd thin film contains a larger amount of Pd of these two films, and it is therefore considered that the comparison is not particularly against the hydrogen sensor using the Pd thin film.

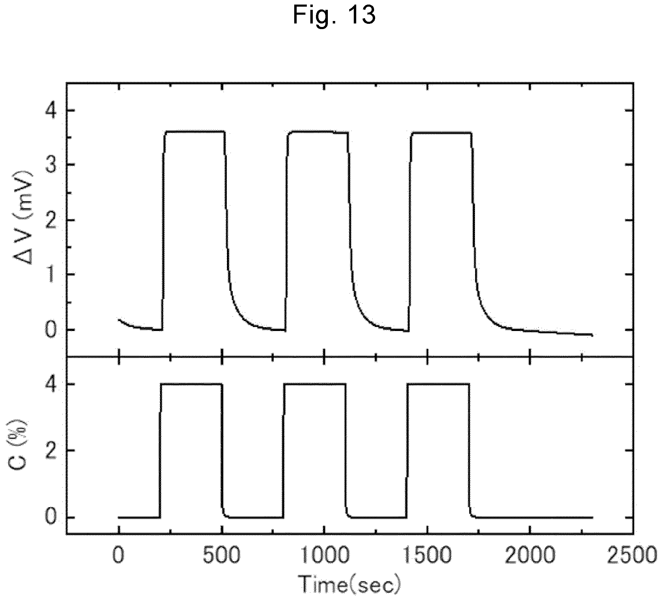

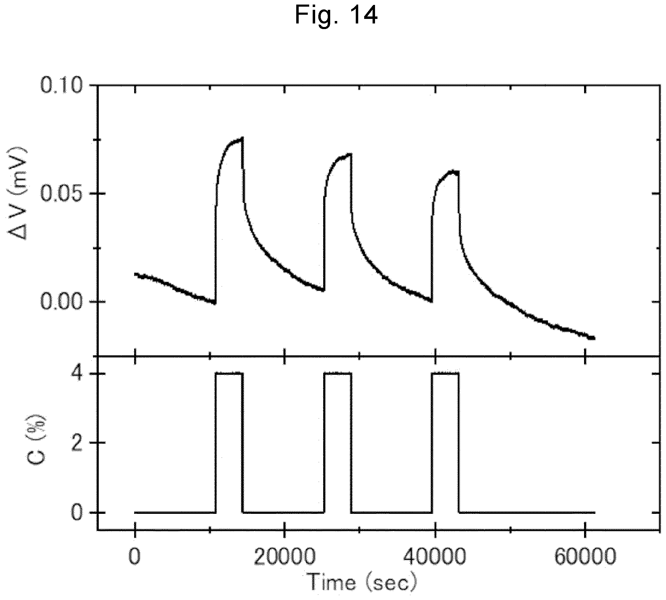

In the comparative experiment, a nitrogen gas in which 4% of hydrogen is mixed and a pure nitrogen gas were repeatedly introduced into the two hydrogen sensors mentioned above to measure sensor outputs. Data of the hydrogen sensor of the example of the present invention and the hydrogen sensor using the Pd thin film are shown in the graphs in Figs. 13 and 14, respectively. In these figures, the lower side shows changes in hydrogen concentration (%) in the nitrogen gas introduced into the hydrogen sensor, and the upper side shows changes in output voltage (mV) of the hydrogen sensor. The switching of gases was performed every five minutes in the hydrogen sensor of the example of the present invention and every 180 minutes in the hydrogen sensor using the Pd thin film. The reason for which they have different switching periods is that the two sensors have different speeds of response to hydrogen gas. Specifically, it took about 10 seconds for the hydrogen sensor of the example of the present invention and about 60 minutes for the hydrogen sensor using the Pd thin film to reach a saturation value after the hydrogen-mixed nitrogen gas was introduced. In addition, it can be seen from the repeated peak heights of the output signals of the hydrogen sensors that the hydrogen sensor of the example of the present invention has higher output voltage and also approximately constant peak heights despite repeated introduction, while for the hydrogen sensor using the Pd thin film, the peak heights decrease with repeated introduction, which means poor reproducibility.

As a result of the above comparison, the hydrogen sensor of the example of the present invention has a larger response speed, better reproducibility (lower hysteresis), and higher signal intensities, and this result is associated with the fact that Pd in PdCuSi is in the form of minute particles and in the state of alloy and thus exhibits good adhesion to the MSS substrate.

### Industrial Applicability

As described above, the present invention is expected to have high industrial applicability such as availability for ensuring high safety of a fuel cell, hydrogen-related facilities or the like by using a hydrogen sensor with a simple configuration.

### Citation List

### Patent Literature

Patent Literature 1: JP 2015-45657 A
Patent Literature 2: JP WO2013/157581 A1
Patent Literature 3: JP WO2011/148774 A1
Patent Literature 4: JP 2008-8869 A
Patent Literature 5: JP 2009-139106 A
Patent Literature 6: JP 2010-181282 A
Patent Literature 7: JP 2017-215170 A

### Non Patent Literature

Non Patent Literature 1: S. Okuyama et al., Jpn. J. Appl. Phys., 39(2000)3584.
Non Patent Literature 2: Yen-I Chou et al., Tamkang J. Sci. Eng., 10(2007)159.
Non Patent Literature 3: JIS W 0201:1990 Standard Atmosphere
Non Patent Literature 4: Susumu Kajita, Panasonic Technical J. Vol.61 (2015)61.
Non Patent Literature 5: Hiroaki Yamazaki et al., IEEJ Trans. Sens. Micro. 138(2018)312.
Non Patent Literature 6: G. Yoshikawa et al., Nano Lett., 11(2011)1044.
Non Patent Literature 7: G. Yoshikawa et al., Sensors 12(2012)15873.
Non Patent Literature 8: G. Imamura et al., Anal. Sci., 32(2016)1189.
Non Patent Literature 9: G. Yoshikawa, Applied Physics Letters 98, 173502 (2011).

## Claims

1. A hydrogen sensor having a sensitive film on a surface for receiving surface stress of a membrane-type surface stress sensor, **characterized by** that the sensitive film is an amorphous palladium-copper-silicon alloy thin film having a thickness of greater than 0 nm and less than 100 nm, and wherein the membrane-type surface stress sensor comprises:
a thin plate-shaped member with four peripheral portions thereof supported from outside to provide four support portions, wherein the thin plate-shaped member and the four peripheral portions are made from silicon, the thin plate-shaped member has a disc shape or a shape having symmetry of rotation by an integer multiple of 90 degrees about its center, and the four support portions are provided at positions of rotational symmetry at 90 degrees from the center of the thin plate-shaped member, and
four stress detecting parts provided at the four support portions by forming piezo-resistive elements, wherein four resistances of the four stress detecting parts are connected in a full Wheatstone bridge configuration.

2. The hydrogen sensor as claimed in claim 1, wherein an atomic ratio between palladium, copper, and silicon contained in the amorphous palladium-copper-silicon alloy is 65 < x < 90, 3 < y < 20, and 3 < z < 20 wherein the alloy is represented as PdₓCu_{y}Si_{z} with x, y, and z being percentage values.

3. The hydrogen sensor as claimed in claim 1 or 2, wherein the sensitive film has a thickness of greater than 0 nm and 50 nm or less.

4. The hydrogen sensor as claimed in claim 3, wherein the sensitive film has a thickness of 15 nm or more and 50 nm or less.

5. The hydrogen sensor as claimed in claim 3, wherein the sensitive film has a thickness of 5 nm or more and 50 nm or less.

6. A method for detecting hydrogen comprising alternately supplying a target gas containing hydrogen and a purge gas to the hydrogen sensor as claimed in any one of claims 1 to 5 and measuring a hydrogen concentration in the target gas based on an output signal from the hydrogen sensor.

7. The hydrogen detecting method as claimed in claim 6, comprising performing an arithmetic treatment on the output signal.

8. The hydrogen detecting method as claimed in claim 7, wherein the arithmetic treatment is time differentiation.

9. The hydrogen detecting method as claimed in claim 8, comprising performing a process of calculating the hydrogen concentration based on a peak value of the output signal on which the time differentiation has been performed.

10. The hydrogen detecting method as claimed in claim 8, comprising performing a process of obtaining the hydrogen concentration based on a signal waveform following a peak value of the time differentiated output signal.

11. The hydrogen detecting method as claimed in claim 10, wherein the process of obtaining the hydrogen concentration based on the signal waveform following the peak value of the time differentiated output signal is selectively performed based on the peak value of the time differentiated output signal.

## Patentansprüche

1. Wasserstoffsensor, der einen empfindlichen Film auf einer Oberfläche zum Empfangen von Oberflächenspannung auf einem Membran-Oberflächenspannungssensor aufweist, **dadurch gekennzeichnet, dass** der empfindliche Film ein Dünnfilm aus amorpher Palladium-Kupfer-Silizium-Legierung ist, der eine Dicke größer 0 nm und geringer als 100 nm aufweist, und wobei der Membran-Oberflächenspannungssensor Folgendes umfasst:
ein dünnes, plattenförmiges Bauteil, wobei vier Randabschnitte davon von außen gestützt werden, um vier Stützabschnitte bereitzustellen, wobei das dünne, plattenförmige Bauteil und die vier Randabschnitte aus Silizium hergestellt sind, das dünne, plattenförmige Bauteil eine Scheibenform oder eine Form aufweist, die Drehsymmetrie um ein ganzzahliges Vielfaches von 90 Grad um seinen Mittelpunkt aufweist, und die vier Stützabschnitte bei Positionen der Drehsymmetrie bei 90 Grad von dem Mittelpunkt des dünnen, plattenförmigen Bauteils bereitgestellt sind, und
vier Spannungserfassungsteile, die an den vier Stützabschnitten bereitgestellt werden, indem piezoresistive Elemente gebildet werden, wobei vier Widerstände der vier Spannungserfassungsteile in einer Wheatstoneschen Vollbrückenkonfiguration verbunden sind.

2. Wasserstoffsensor nach Anspruch 1, wobei ein Atomverhältnis zwischen Palladium, Kupfer und Silizium, die in der amorphen Palladium-Kupfer-Silizium-Legierung enthalten sind, 65 < x < 90, 3 < y < 20 und 3 < z < 20 ist, wobei die Legierung als PdₓCu_{y}Si_{z} dargestellt wird, wobei x, y und z Prozentwerte sind.

3. Wasserstoffsensor nach Anspruch 1 oder 2, wobei der empfindliche Film eine Dicke größer 0 nm und von 50 nm oder weniger aufweist.

4. Wasserstoffsensor nach Anspruch 3, wobei der empfindliche Film eine Dicke von 15 nmoder mehr und 50 nm oder weniger aufweist.

5. Wasserstoffsensor nach Anspruch 3, wobei der empfindliche Film eine Dicke von 5 nm oder mehr und 50 nm oder weniger aufweist.

6. Verfahren zum Erfassen von Wasserstoff, umfassend abwechselndes Zuführen eines Zielgases, das Wasserstoff enthält, und eines Spülgases zu dem Wasserstoffsensor nach einem der Ansprüche 1 bis 5 und Messen einer Wasserstoffkonzentration in dem Zielgas, basierend auf einem Ausgangssignal des Wasserstoffsensors.

7. Wasserstofferfassungsverfahren nach Anspruch 6, umfassend das Durchführen einer arithmetischen Behandlung des Ausgangssignals.

8. Wasserstofferfassungsverfahren nach Anspruch 7, wobei die arithmetische Behandlung eine Zeitdifferenzierung ist.

9. Wasserstofferfassungsverfahren nach Anspruch 8, umfassend das Durchführen eines Prozesses zum Berechnen der Wasserstoffkonzentration basierend auf einem Spitzenwert des Ausgangssignals, an dem die Zeitdifferenzierung durchgeführt worden ist.

10. Wasserstofferfassungsverfahren nach Anspruch 8, umfassend das Durchführen eines Prozesses zum Erhalten der Wasserstoffkonzentration basierend auf einer Signalwellenform, die einem Spitzenwert des zeitdifferenzierten Ausgangssignals folgt.

11. Wasserstofferfassungsverfahren nach Anspruch 10, wobei der Prozess zum Erhalten der Wasserstoffkonzentration basierend auf der Signalwellenform, die dem Spitzenwert des zeitdifferenzierten Ausgangssignals folgt, selektiv basierend auf dem Spitzenwert des zeitdifferenzierten Ausgangssignals durchgeführt wird.

## Revendications

1. Capteur d'hydrogène présentant un film sensible sur une surface pour recevoir la contrainte de surface d'un capteur de contrainte de surface de type à membrane, **caractérisé en ce que** le film sensible est un film mince d'alliage amorphe de palladium-cuivre-silicium présentant une épaisseur supérieure à 0 nm et inférieure à 100 nm, et dans lequel le capteur de contrainte de surface de type à membrane comprend :
un élément en forme de plaque mince dont quatre portions périphériques sont soutenues de l'extérieur pour fournir quatre portions de support, dans lequel l'élément en forme de plaque mince et les quatre portions périphériques sont en silicium, l'élément en forme de plaque mince présente une forme de disque ou une forme présentant une symétrie de rotation d'un multiple entier de 90 degrés par rapport à son centre, et les quatre portions de support sont fournies en des positions de symétrie de rotation à 90 degrés par rapport au centre de l'élément en forme de plaque mince, et
quatre parties de détection de contrainte fournies aux quatre portions de support en formant des éléments piézorésistifs, dans lequel les quatre résistances des quatre parties de détection de contrainte sont reliées dans une configuration de pont de Wheatstone complet.

2. Capteur d'hydrogène selon la revendication 1, dans lequel un rapport atomique entre le palladium, le cuivre et le silicium contenus dans l'alliage amorphe de palladium-cuivre-silicium est de 65 < x < 90, 3 < y < 20, et 3 < z < 20, dans lequel l'alliage est représenté par PdₓCu_{y}Si_{z}, x, y et z étant des valeurs en pourcentage.

3. Capteur d'hydrogène selon la revendication 1 ou 2, dans lequel le film sensible présente une épaisseur supérieure à 0 nm et inférieure ou égale à 50 nm.

4. Capteur d'hydrogène selon la revendication 3, dans lequel le film sensible présente une épaisseur supérieure ou égale à 15 nm et inférieure ou égale à 50 nm.

5. Capteur d'hydrogène selon la revendication 3, dans lequel le film sensible présente une épaisseur supérieure ou égale à 5 nm et inférieure ou égale à 50 nm.

6. Procédé de détection d'hydrogène comprenant l'alimentation alternée en gaz cible contenant de l'hydrogène et en gaz de purge au capteur d'hydrogène selon l'une quelconque des revendications 1 à 5 et la mesure d'une concentration en hydrogène dans le gaz cible sur la base d'un signal de sortie provenant du capteur d'hydrogène.

7. Procédé de détection d'hydrogène selon la revendication 6, comprenant la réalisation d'un traitement arithmétique sur le signal de sortie.

8. Procédé de détection d'hydrogène selon la revendication 7, dans lequel le traitement arithmétique est une différenciation temporelle.

9. Procédé de détection d'hydrogène selon la revendication 8, comprenant la réalisation d'un processus de calcul de la concentration en hydrogène sur la base d'une valeur de crête du signal de sortie sur lequel la différenciation temporelle a été effectuée.

10. Procédé de détection d'hydrogène selon la revendication 8, comprenant la réalisation d'un processus d'obtention de la concentration en hydrogène sur la base d'une forme d'onde de signal suivant une valeur de crête du signal de sortie différencié dans le temps.

11. Procédé de détection d'hydrogène selon la revendication 10, dans lequel le processus d'obtention de la concentration en hydrogène sur la base de la forme d'onde de signal suivant la valeur de crête du signal de sortie différencié dans le temps est sélectivement effectué sur la base de la valeur de crête du signal de sortie différencié dans le temps.
